(19) Europäisches Patentamt
European Patent Office
Office européen des brevets

(11) **EP 3 210 558 B1**

(12) **EUROPEAN PATENT SPECIFICATION**

(45) Date of publication and mention
of the grant of the patent:
**09.05.2018 Bulletin 2018/19**

(51) Int Cl.:
*A61B 18/14* $^{(2006.01)}$  *A61B 18/00* $^{(2006.01)}$
*A61B 18/08* $^{(2006.01)}$

(21) Application number: **17151043.1**

(22) Date of filing: **11.01.2017**

(54) **MEDICAL DEVICE**

MEDIZINISCHE VORRICHTUNG

DISPOSITIF MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.01.2016 US 201662279188 P**

(43) Date of publication of application:
**30.08.2017 Bulletin 2017/35**

(73) Proprietor: **Cook Medical Technologies LLC
Bloomington, IN 47404 (US)**

(72) Inventors:
• **ELGAARD, Per
4690 Haslev (DK)**
• **PAAMAND, Rune Tore
2700 Broenshoej (DK)**

• **TORP, Allan
4632 Bjaeverskov (DK)**
• **BUTZBACKER, Raimo Urban
4690 Haslev (DK)**

(74) Representative: **Mathys & Squire LLP
The Shard
32 London Bridge Street
London SE1 9SG (GB)**

(56) References cited:
**EP-A1- 0 707 830    EP-A1- 0 752 235
EP-A2- 2 939 629    US-A- 6 110 168
US-A- 6 149 681     US-A1- 2010 191 151
US-B2- 8 852 178**

**Description**

FIELD

The present disclosure relates generally to medical devices. More specifically, the disclosure relates to a device and method(s) for occluding or closing a body vessel using a radiofrequency signal and a current to heat and/or ablate the body vessel.

BACKGROUND

There are numerous medical conditions when it is desired or necessary to close a body vessel, including the treatment of aneurysms, arteriovenous malformations, arteriovenous fistulas, for starving organs of oxygen and nutrients, in the treatment or containment of cancerous growths, and so on.

Several techniques are known and in use for closing or occluding such body vessels. Traditionally, vessels have been closed by means of external ligation, which generally must be carried out by an open surgery procedure, with its associated risks, inconvenience, and long patient recovery times. Other, more recent, methods aim to use an endoluminal procedure to insert into the vessel or organ one or more occlusion devices, such as a metal framed occluder, coils, pellets or the like, able to obstruct the flow of blood in the vessel.

It is also known to seek to constrict a vessel by endoluminal ablation, causing contraction of the vessel and/or coagulation of blood to form a blood clot in the vessel. Various methods can be employed to cause such ablation. Document EP0707830 A1 discloses a device for occlusion of a vessel including an AC and DC power supply. The system first uses AC power (at RF frequencies) to occlude the vessel. In a second step DC current is applied to to detach the coil from the catheter.

EP2939629 A2, discloses a two-step process for occluding a blood vessel, where energy is applied in a first step until a first threshold is met and then energy is applied at a second lower level until a second threshold is met.

BRIEF SUMMARY

The invention may include any of the following embodiments in various combinations and may also include any other aspect described below in the written description or in the attached drawings. This disclosure provides a medical device and methods for conducting vessel ablation and occlusion. The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes.

The device may be used to heat a body vessel, and it may have a support including a proximal end and extending to a distal end, the support defining a longitudinal axis. The device may optionally include a coil disposed about the support and being electrically conductive. The conductive element or coil may be coated with a dielectric material in order to have a low-friction tip. The dielectric material may be a polymer, in particular parylene C. The thickness of the coating may range from 1.5 nanometers to 1 micron thick. The coating may extend across the entirety of the conductive element, or only a portion of the conductive element, particularly the distal-most fraction. The coil may have a first end being disposed between the proximal and distal ends, the coil extending from the first end to a second end disposed at the distal end.

The device may further include a control unit being operatively coupled to the first end and the second end, the control unit having a signal generator and a power supply. The signal generator may be operable to transmit a radiofrequency signal from the coil to the body vessel when the device is in a radiofrequency mode. The resistive part may be at a first temperature in the radiofrequency mode. The power supply may be operable to transmit a current through the coil when the device is in a resistive mode. The current may heat the resistive part to a second temperature being greater than the first temperature in the resistive mode. The second temperature may be sufficient to occlude and swell the body vessel.

The device may be part of an assembly having the features discussed above, and also having an outer sheath having a first sheath end and extending to a second sheath end. The outer sheath may form an inner lumen therethrough from the first sheath end to the second sheath end such that the support is slidably disposed within the inner lumen.

The device may comprise an occlusion member may include a balloon catheter with an inflatable element. The balloon catheter may include a catheter body having a first end and extending to a second end, the catheter body defining a lumen therethrough, the lumen having a diameter sized to fit the support coaxially therein. The coil of the device may extend distal of the second end of the catheter body.

A method of occlusion and ablation is also described. A method of use of the device may include (1) positioning a device in the body vessel with the conductive member or coil positioned at a treatment site; (2) occluding the body vessel upstream or downstream of the treatment site, such as with the inflatable member of a balloon catheter, to stop or minimize blood flow; (3) transmitting a radiofrequency ("RF") signal from the coil to a return electrode to heat the body vessel, the device being in the radiofrequency mode; (4) detecting at least one change in the coil; and (5) transmitting a current through the coil when the at least one change is detected to heat the body vessel, the device being in the resistive mode. In this method, the device may be provided integrally or separately from the balloon catheter. In one embodiment, the balloon catheter may be introduced percutaneously at a first upstream site and the device may be introduced percutaneously at a second downstream site, closer to the treatment site. In some embodiments, the device may be positioned coaxially with an in a lumen of a balloon catheter.

Various additional features and embodiments will become apparent with the following description. The present disclosure may be better understood by referencing the accompanying figures.

BRIEF DESCRIPTION OF THE DRAWINGS

Fig. 1 depicts a partial, environmental view of a medical device in accordance with one embodiment of the present disclosure;
Fig. 2A depicts a cross-sectional, side view of the device of Fig. 1;
Fig. 2B depicts a partial, blown-up, side view of the device of Fig. 1;
Fig. 3 depicts a side view of the device of Fig. 1;
Fig. 4 depicts a side view of the device of Fig. 1 in a radiofrequency mode;
Fig. 5 depicts a side view of the device of Fig. 1 in a resistive mode;
Fig. 6 depicts steps of a method of use of the device of Fig. 1 in accordance with one embodiment of the present disclosure;
Fig. 7 depicts steps of another method of use of the device of Fig. 1 in accordance with another embodiment of the present disclosure; and
Fig. 8 is a side view of the distal ends of devices in accordance with another embodiment of the present disclosure.

DETAILED DESCRIPTION

The present disclosure will now be described more fully with reference to the accompanying figures, which show various embodiments. The accompanying figures are provided for general understanding of various embodiments and method steps. However, this disclosure may be embodied in many different forms. These figures should not be construed as limiting, and they are not necessarily to scale.

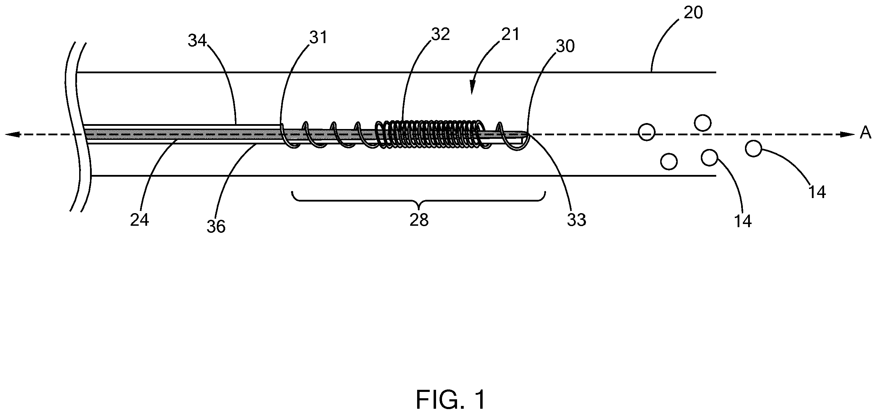

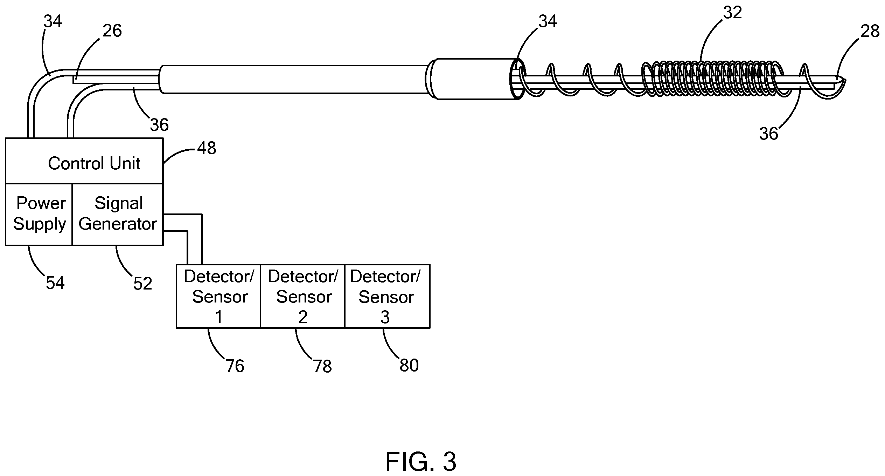

Fig. 1 depicts an environmental view of one embodiment of the medical device that may be used to heat a body vessel at or adjacent a treatment site 21. The body vessel has a vessel wall 20. In this view, the device may be placed or positioned in the body vessel in a body. The device may have a support 24, or mandrel, that extends from a proximal end (depicted in Fig. 3 (26)) to a distal end 30. The support 24 may define a longitudinal axis A.

A coil 32 may be disposed about the support 24, and the coil 32 may be electrically conductive. The coil 32 may have a first end 31 being disposed between the proximal and distal ends (26, 30), and the coil 32 may extend to a second end 33 being disposed at the distal end 30. The support 24 may have a distal segment 28 that supports the coil 32, or about which the coil 32 is disposed.

The distal segment 28 may have an outer diameter that distally decreases to form a distal taper (as shown in Fig. 1). Alternatively, the support 24, including its distal segment 28, may have an outer diameter being uniform from the proximal end 26 to the distal end 30. The distal taper or tapered tip may provide the advantage of making the distal end 30 easier to track within the body vessel. Such distal taper may provide flexibility to track the device. In another embodiment, the distal portion of the device may have curvature similar to guidewires known in the art, which confers similar advantages to a distal taper.

The device may further have a first wire 34 and a second wire 36. The first wire 34 may be electrically coupled or connected to a control unit (depicted in Fig. 3 (48)). The first wire 34 may extend from the control unit and along the longitudinal axis A to the first end 31. The first wire 34 may be attached to the first end 31 such that the connection provides an electrical coupling between the first wire 34 and the coil 32.

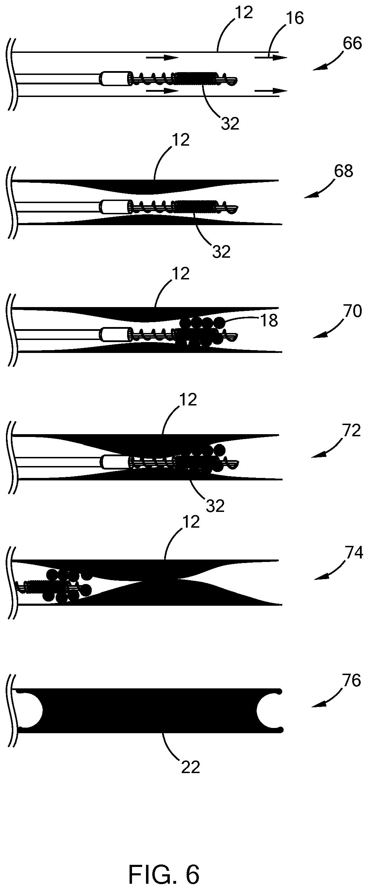

The second wire 36 may also be electrically coupled or connected to the control unit and extend from the control unit, along the longitudinal axis A, to the second end 30. The second wire 36 may be attached to the second end 33 such that the connection provides an electrical coupling between the second wire 36 and the coil 32. In this way, the device may form a first circuit along the path created by the control unit, the first wire 34, the coil 32, and the second wire 36. The device may form various circuits, which will be discussed further in Figs. 3-5. In Fig. 1, coagulated blood 14 may start to form as the device operates, which will also be discussed further in Fig. 6.

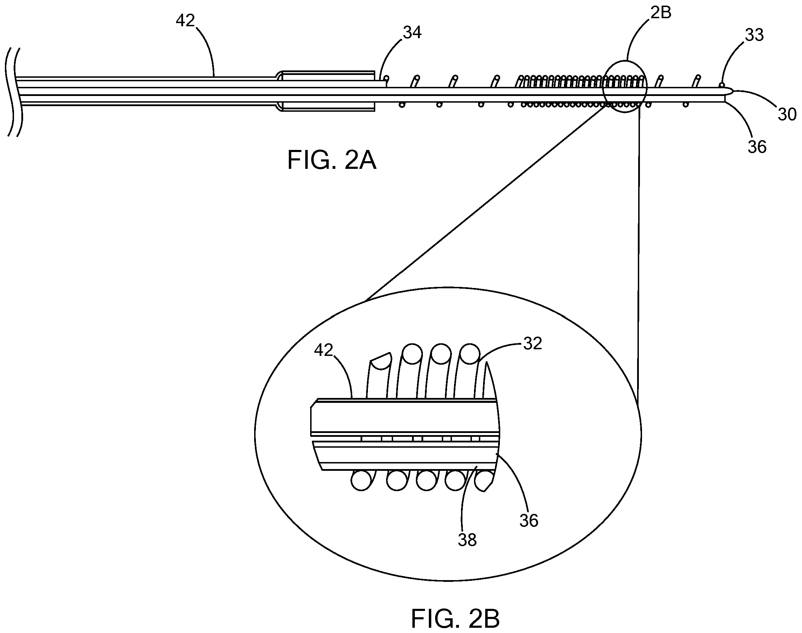

Figs. 2A-B depicts further details of the device. For example, Fig. 2A shows a cross-sectional view of the device. Fig. 2B shows a blown-up view of the device around circle 2B. In Fig. 2A, the support 24 has a uniform outer diameter from the proximal end to the distal end 30. Additionally, either or both of the first and second wires (34, 36) may have an insulator disposed about their outer surface to electrically insulate them from the rest of the device. Insulator 38 is disposed about the second wire 36 in Fig. 2B.

Additionally, the device may have a shrink tubing disposed about the device. For example in Fig. 2A, the shrink tubing 42 may extend around the support 24, the first wire 34, the second wire 36, and/or any other portion of the device. As one advantage, the shrink tubing 42 may immobilize or bind the support 24, the first wire 34, and the second wire 36 in place so that they are immobilized relative to each other. The shrink tubing 42 may also immobilize and/or extend over a part of the coil 32 to keep the coil 32 in position as the device is in use. Alternatively or additionally in Fig. 2B, the shrink tubing 42 may only be disposed about the support 24. In this configuration, the shrink tubing 42 may act to isolate or insulate the support 24 from the rest of the device.

Fig. 3 further depicts the proximal end of the device. The proximal end may include a control unit 48 being operatively coupled or connected to the first end of the coil 32 by way of the first wire 34. Additionally, the control unit 48 may be operatively coupled to the second end of the coil 32 by way of the second wire 36. The control unit 48 may be coupled to or include a signal generator 52 to generate a radiofrequency signal and a power supply 54 to generate a current. The power supply could be a battery and/or other forms of direct current or alternating current.

The signal generator 52 may be operable by the control unit 48 and the user to transmit the RF signal from the coil and to the body vessel when the device is in a radiofrequency mode. In one embodiment, the RF signals may be an alternating current (AC) signal having a magnitude from about 10 kilohertz to about 1 megahertz. The power supply 54 may be operable by the control unit 48 and the user to transmit a current through the coil when the device is in a resistive mode. These modes of the device, and how they contribute to heating the body vessel, will be discussed in further detail below. In the resistive mode, the current may be a direct current and/or an alternating current.

In either mode, the amount of current may vary over time, and may depend on the required power to maintain a desired temperature of the coil.

[0033] Power = Current x Voltage     Voltage = Current x Resistance

A device that operates in both RF and resistive heating modes may start with a resistivity of about 50 ohms and a current of about 0.50 amps in the RF mode. After charring occurs, the conductance of the coil will be limited. At this point, the device may switch into resistive heating mode, having a resistivity of about 120 ohms and a current of about 0.33 amps. Charring on the coil may create an increase in overall resistance and/or impedance of the electrical circuits in the device.

In the RF mode, the RF signal generates a field around the tip. The RF signal may not deliver enough power to heat the coil itself. Rather, the coil is maintained at a first temperature (e.g. body temperature). In the resistive mode, the power delivered may increase and be great enough to heat the resistive part (e.g. resistive tip at second end 33) to a target temperature, or second temperature being greater than the first temperature, such that the target temperature is sufficient to inflame or occlude the body vessel. In this way, the coil may be at a first temperature in RF mode and at a second, higher temperature in the resistive heating mode. The second temperature may be just above human body temperature or much greater than human body temperature.

The control unit 48 may also include an electrode drive unit (not shown) for moving the coil within the patient's vessel. This may also be done manually. In some embodiments, the control unit 48 may have or be coupled to a plurality of sensors and/or detectors (76, 78, 80) to determine different conditions of the device. For example, the plurality of sensors may be sensors selected from the group consisting of temperature sensors, current sensors, timers, impedance sensors, and pressure sensors. These various sensors may be used to detect and determine temperature, current, time, impedance, and/or resistance, respectively, to assist the user in using the device as the active components may not be visually accessible to the user.

The control unit 48 may optionally include a user interface coupled to the control unit 48 and operable to provide data to the user and for input of user commands to the control unit 48. The user interface may, in its simplest embodiment, include an on/off switch for operating the control unit 48, and ablation and/or coagulation, with the control unit 48 then effecting the desired ablation process under the command of the control unit 48. In other embodiments, the user interface may be more sophisticated and enable, for example, a user to select different modes of ablation and optionally to produce, for instance, occluding barriers of different lengths, sizes, or lengths and sizes.

The user interface also may have an output for providing ablation feedback and/or warning signals to a user. It may, for example, provide an indication of measured temperature and/or impedance, an indication of progress of ablation of the vessel and so on. For such purposes, the user interface may include a visual unit, for example a display to display quantitative data such as graphs, measures of temperature and impedance, determined length of occlusion and so on. In other embodiments, the display may be simpler, having for instance simple visual indicators such as one or more illuminated lamps. The output could also be an acoustic output and/or, as appropriate, a tactile output such as a vibration generator and so on. Any combination of user feedback devices may be provided.

When in use, the device may operate in two modes: (1) a RF mode for RF heating and/or ablation and (2) a resistive heating mode for resistive heating and ablation. Both modes may use the coil 32 to create a closed loop or circuit. In one or both modes, the device is designed to create blood clotting, that is to ablate the blood surrounding the electrical element. This can be achieved by selecting an ablation energy level and an ablation time duration suitable to heat surrounding blood, which in some circumstances can be expected to be less than the energy required to ablate the vessel tunica (e.g. tunica externa), although there may be experienced some contraction of the vessel as a result of the heating of the blood. The skilled person will be able to determine suitable ablation parameters from common general knowledge in the art.

It is to be appreciated that the level of power applied through the electrode and the time of application will be dependent upon factors including the size of the vessel, the amount and speed blood flow through the vessel, pulsation and turbulence of blood at the point of ablation, and so on.

In Fig. 3, elements of the device may create various circuits. For example, Fig. 3 depicts elements that form a first circuit or electrical pathway for use in the resistive mode. Specifically, the first wire 34 may be electrically coupled to the control unit 48 and extend from the control unit 48 and along the longitudinal axis to the first end of the coil 32. The first wire 34 may be attached to the first end of the coil 32. The second wire 36 may also be electrically coupled to the control unit 48 and extend from the control unit 48 and along the longitudinal axis to the second end of the coil 32. The second wire 36 may be attached to the second end of the coil 32. The control unit 48, the first wire 34, the coil 32, and a second wire 36 form the first circuit being operable in the resistive mode. Further details of the RF and resistive modes will be discussed below in turn.

RF Mode

Generally in the RF mode, an electrical terminal is fed endoluminally into the vessel and an electrical pulse and/or constant electrical signal at RF frequencies applied to the electrical terminal. The conductivity of blood and/or the vessel tissues causes localized heating of the blood and tissue and not significant heating of the resistive part of the coil itself, creating a local zone that is heated by the RF frequencies. This heating can be used to cause damage to the tissue (intima) of the vessel wall, resulting in vessel contraction. In other devices, RF ablation heats the surrounding blood, causing this to coagulate around the electrical terminal and form a blood clot which blocks the vessel.

Two types of RF ablation apparatus are generally contemplated in the art: a monopolar system and a bipolar system. A monopolar system may have an elongate first electrode (e.g. active electrode) and a second electrode (e.g. dispersive electrode) pad or return electrode positioned outside the patient's body. The anode terminal is designed to be fed endoluminally into the patient's vessel, while the cathode pad is positioned against the person's outer body, as close as practicable to the anode terminal. Electrical energy applied to the anode terminal will pass by conduction through the patient to the cathode pad. There will be localized heating at the anode terminal, which effects the desired ablation.

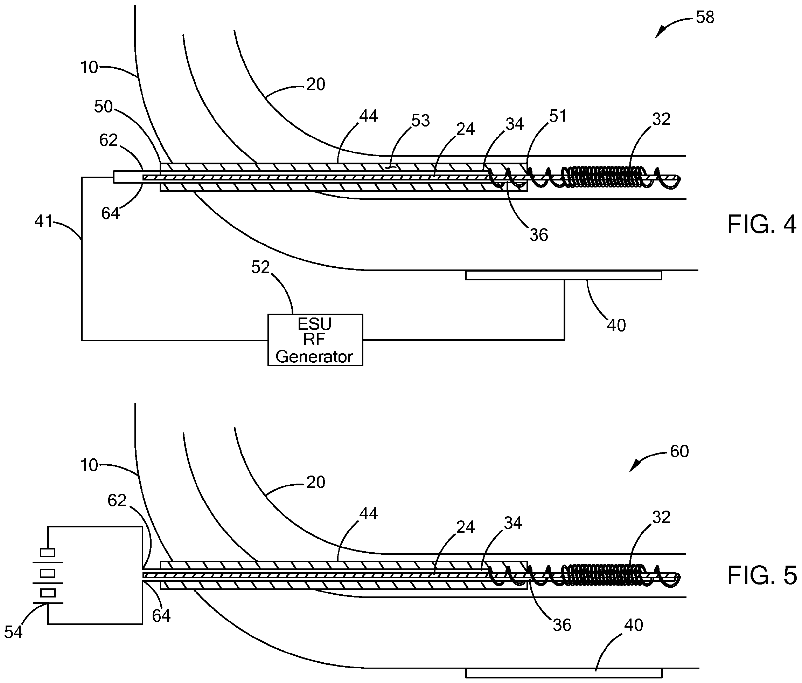

Fig. 4 depicts details of the device in the RF mode 58. A pad or return electrode 40 may be arranged in various forms having various circuits. For example, in a monopolar system or mode, the return electrode 40 may be electrically coupled to the control unit (e.g. RF generator 52) and disposable outside of the body 10. In this example, the RF signal generator 52, the first wire 34, the coil 32, and return electrode 40 may form a second circuit for the RF signal. The RF frequencies, and the power they generate,may not be large enough to heat the first electrode and/or the return electrode. However, the RF signal and field generated will cause localized heating of the surrounding blood and tissue. The temperature of the first electrode remains at a first temperature (e.g. body temperature).

In the monopolar mode, the radiofrequency signal may be transmitted through the second circuit having the RF signal generator 52, the first wire 34, the coil 32, and the second wire 36 including the return electrode 40. Advantageously, the monopolar system may be easier to manufacture, having less elements immobilized or attached to the support 24.

The RF mode 58 may also operate in a bipolar system. In a bipolar system, the return electrode 40 may be disposed within the second wire 36 (similarly to the first circuit of the resistive mode discussed with Fig. 3). In this way, the signal generator 52, the first wire 34, the coil 32, and a return electrode 40 being part of the second wire 36 form a third circuit for the RF signal.

In the bipolar mode, the radiofrequency signal may be transmitted through the third circuit having the RF signal generator 52, the first wire 34, the coil 32, and the second wire 36. Advantageously, operating the device having a bipolar system may be easier for the user by not having a separable return electrode disposable outside of the body 10.

Using either the second circuit (monopolar system) or the third circuit (bipolar system), the device may transmit an RF signal from the coil 32 to the return electrode 40, heating the body vessel when in the RF mode. A method step of transmitting a RF signal may include transmitting the RF signal with the monopolar mode or the bipolar mode.

The first wire 34 may be attached via a first lead 62 and a conductive wire 41 to the signal generator 52. Additionally, the second wire 36 may be attached to the signal generator 52 through a second lead 64 and the conductive wire 41. One skilled in the art will understand that various conductive wires and connectors may be used to electrically couple parts of the device.

The device may optionally include an outer sheath 44 for delivery and/or retrieval. The outer sheath 44 may optionally have a first sheath end 50 and extend to a second sheath end 51. The outer sheath 44 may form an inner lumen 53 therethrough from the first sheath end 50 to the second sheath end 51. As shown in Fig. 4, the support 24 may be slidably disposed or received within the inner lumen 53.

## Resistive Heating Mode

The resistive part of the coil 32 has a higher electrical resistance than the other parts of the electrically conductive element (e.g. 50-200 ohms). In this embodiment, the resistive part is the operative part of the device. The resistive part is configured so that the application of power to the wires (34, 36) causes current to flow through the resistive part, which can cause heating of the resistive part to a second temperature being greater than body temperature. This, in turn, causes embolization and/or ablation of blood surrounding the resistive part, and/or heating and consequential contraction of the vessel in the vicinity of the resistive part through the thermal energy at the resistive part. Structurally, the resistive part of the coil could be any part of the coil. In one example, the resistive part is the second end 33.

Fig. 5 depicts the resistive heating mode or resistive mode 60. In a method of use of the device, after transmitting the RF signal, the control unit and/or a sensor may detect at least one change in the coil 32. Once this occurs, the control unit may switch the device to the resistive mode 60.

This change may be a change in impedance. Impedance is a measure of the amount of opposition that a part of a circuit may present to a current. For example, if part of a circuit is more resistive, the impedance indicates the level of resistance. If part of a circuit becomes more resistive over time, a change in impedance can indicate that corresponding change in resistance.

Because of this, a change in resistance is a suitable parameter to measure to determine when to switch the device from the RF mode to the resistive mode. With RF signal, over time the blood may start to char in the local zone of the RF signal. This charring may cause the blood to coagulate on the coil, inhibiting its ability to deliver a sufficient RF signal for vessel ablation. Once this change is detected, the device may switch to the resistive mode. This may involve transmitting a current through the coil 32 when the at least one change is detected to heat the body vessel and the vessel wall 20.

As described above in Fig. 3, in the resistive mode 60 a power supply 54 may generate a current to transmit through the coil 32. The current may be transmitted through the first circuit, including the power supply 54, the first wire 34, the coil 32, and the second wire 36. As the current flows through the higher resistance part of the coil 32, the coil heats up. This may heat the vessel wall 20, causing further coagulation and occlusion than already occurred with the RF signal. Having these two modes and corresponding structures in one device allows a user to have the advantages of RF heating and resistive heating to occlude a body vessel.

Additionally or alternatively, the resistive mode 60 may involve using the RF generator. In this case, the resistive mode 60 may not need or use the power supply. Instead, once blood begins to char on the coil and isolate it from the body vessel, the RF signal will primarily heat the electrode resistively. This will automatically start the resistive mode 60 based on the overall change in resistance and/or impedance in the overall circuit. The balance between the RF mode and the resistive mode can be measured with a resistivity sensor. A constant power output can be maintained by adjusting a voltage source.

Returning to Fig. 5, the first wire 34 may be attached via a first lead 62 to a power supply 54 (e.g. a battery). Additionally, the second wire 36 may be attached to the power supply 54 through a second lead 64. Various conductive wires and connectors may be used to electrically couple parts of the device.

Fig. 6 depicts a method to fully occlude a body vessel 12. In step 66, the device may be positioned in the body vessel 12 adjacent the treatment site. At this time, blood flow 16 will be substantially normal. In step 68, the device may generate the RF signal with a signal generator. The RF signal may be transmitted with a monopolar system or a bipolar system. With the monopolar system, the RF signal may flow through the second circuit. In the bipolar mode, the RF signal may flow through the third circuit.

When the RF signal is transmitted, the method may include heating a local zone of the body vessel 12 after the step of transmitting the RF signal. This step of heating may cause swelling of the body vessel 12 at the treatment site, as depicted in step 68. Advantageously, the coil itself may maintain a first temperature near body temperature. As shown, the step of transmitting the RF signal may comprise avoiding contact of the coil 32 with the vessel wall 12.

In step 70, charred blood 18 may start to coagulate and occlude the function of the coil in the RF mode. As the charred blood 18 coagulates on the coil, the device may detect at least one change in the coil. This change may be a change in impedance, a change in temperature, a change in time, a change in current, and a change in resistance. If the device (e.g. sensor) detects a change in impedance due to the charred blood 18, the device may shut off the RF signal and switch to the resistive mode.

Due to the charred blood 18, the device may no longer optimally heat and occlude the body vessel 12. In this condition, it may be advantageous to switch the device to a resistive mode. In step 72, the device may now generate the current with the power supply. The current may flow through the first circuit. As the vessel wall 12 swells, the step of transmitting a current through the coil may comprise contacting the vessel wall with the coil in the resistive mode. Because the resistive mode causes the coil itself to raise in temperature, direct contact with the vessel wall 12 may be advantageous. Heating the vessel wall with the current may comprise the heating swelling the vessel wall further.

In step 74, the resistive mode may start to fully occlude the body vessel 12. As such, the device may start to withdraw from the body vessel 12. This withdrawal may be manual or automatic. In step 76, the body vessel has been fully included with occlusion 22. The device may be fully withdrawn.

In some embodiments, the body vessel in which the ablative procedure is taking place may be occluded in order to minimize heat loss from fluid flow through the body vessel. Flowing fluid, such as blood, contributes to a "heat sink" effect during thermal ablation, wherein heated blood is carried away from the treatment site by the natural flow of blood. In turn, higher power levels are used in order to compensate for the loss of efficiency and energy.

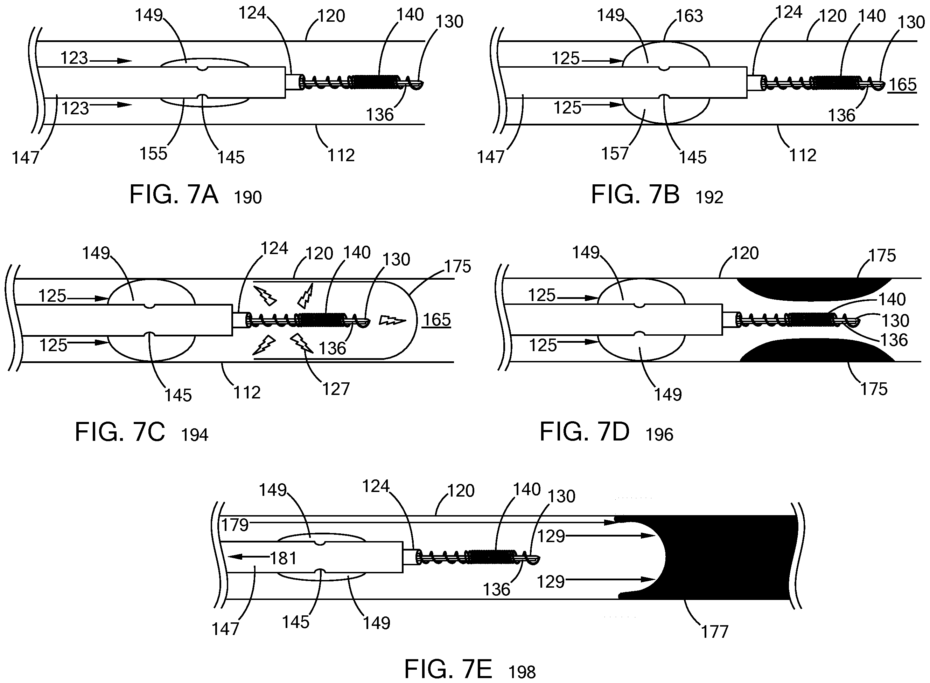

Figs. 7A-7E illustrate a system or device for, and a method of, preventing blood flow through a treatment site at which ablation is to be achieved. The system and method employ an occlusion member of an occlusion device that is deployed upstream or downstream of the treatment site in order to temporarily minimize or prevent the flow of blood through the vessel to be ablated. When the flow of blood is stopped, the ratio of energy that is delivered by the ablation device to the site of ablation is increased, efficiency is increased, and the amount of power that is supplied through the ablation device to the body vessel can be reduced. Such occlusion can also decrease the amount of time required to complete the ablation procedure.

Fig. 7A shows the support 124 of the device arranged within a balloon catheter. The balloon catheter may have a first (proximal) end and may extend to a second (distal) end, and the balloon catheter defines a central lumen formed through catheter body 147 which is sized to accommodate the support of the heating device. In embodiments which include an outer sheath as described above, the central lumen may instead be sized to accommodate the outer sheath. Optionally, a device which incorporates an outer sheath may have the support slidably disposed therein. In one embodiment, the outer sheath may be integrally formed with the balloon catheter. In another embodiment, the outer sheath may be formed as a separate component from the balloon catheter, that is it may be separable from the balloon catheter, or may be manufactured in a separate step, or may be provided as a physically separate component.

The device may include a conductive coil 140, which may be an element such as coil 32 of the preceding figures. The coil winds around, or is disposed around, support 136, is electrically conductive, and has a first coil end disposed between the proximal and distal ends of the support. The coil 140 extends from the first coil end to a second coil end disposed at the distal end 130 of the support 136. The second coil end of coil 140 may be attached, directly or indirectly, to the distal end 130 of support 136, or may be secured to the support 136. In an alternative embodiment, the conductive element may instead be a single electrode.

The coil 140 may extend beyond or distal to the second end of the catheter body 147 and into the lumen of body vessel 120, where the ablation procedure will occur. The catheter body 147 has an expandable balloon or inflatable element 149 attached circumferentially about its exterior. In one embodiment, inflation ports 145 formed through the wall of catheter body 147 allow inflation fluid to flow into and out of the expandable balloon 149. In Fig. 7A, the expandable balloon 149 is in its deflated state 155, thereby permitting the flow of blood 123 through vessel 120.

The device may also include a control unit, such as that described in Fig. 3, with its various circuits, modes of operation, and components.

As shown in Fig. 7A, in a first step 190, the heating device is deployed coaxial with the balloon catheter, fitting into a central lumen of the balloon catheter. In one embodiment, the ablation device (or its support) and the balloon catheter are integral with one another, effectively acting as a single device. In an embodiment in which the ablation device or its support is integral with the balloon catheter, an inflation lumen can run from the proximal end of the balloon catheter through the wall of the catheter for each inflation port 145 provided with the balloon catheter, with the inflation lumen being in fluid communication with the inflation ports. At the first (proximal) end of the catheter body 147, the inflation lumen may be connected to a reservoir of inflation fluid, such that fluid can be introduced into the balloon or withdrawn therefrom, according to the needs at the time of the procedure.

In another embodiment, the balloon catheter and the device are two separate pieces that are assembled together prior to use. In such a case, a commercially-available balloon catheter, such as the Cook Flow-Directed Balloon Catheter (FDB5.3) may be used. In such an embodiment, manufacture may be simplified, and flexibility may be increased for procedures in which occlusion is not necessary.

Fig. 7B shows a second step 192 in which the catheter/ablation device system is positioned at treatment site 165. In this instance, expandable balloon 149 has been inflated to its inflated or expanded state 157. The vessel 120 has now been occluded, with the inflatable element 149 contacting the interior vessel wall at position 163. At this point, the body vessel 120 has been occluded at a position upstream and/or downstream of the treatment site such that the treatment site is substantially free of fluid flow. "Substantially free of fluid flow" in this case means that little or no fluid is passing through the vessel, and that any amount of fluid flow will not be sufficient to disrupt or delay heat-related ablation. As shown in Fig. 7B, blood flow 125 has been impeded, and blood is not able to pass through the occluded vessel due to expanded state 157 of balloon 149.

In a third step 194, Fig. 7C shows the vessel occluded. After occlusion, power is provided to the device, and energy 127 is run through the coil 140. As noted above, energy 127 can be from a number of sources, such as RF signal from a signal generator. The RF signal may be transmitted with a monopolar system or a bipolar system as described above. As shown throughout FIGs. 7A-7E, the step of transmitting the RF signal may comprise avoiding contact of the coil 140 with the vessel wall 112. In other embdomients, contact with the vessel wall in either the RF or the resistive mode may be desired. Charred blood may coagulate on the coil, and the device may detect (such as via a sensor) at least one change in the coil, such as a change in impedance, a change in temperature, a change in time, a change in current, and a change in resistance. If the device (e.g. sensor) detects a change in impedance, the device may shut off the RF signal and switch to the resistive mode. In such a case, the device may generate the current with the power supply. Heat generated by the flow of electric current through a coil when the device is in resistive heating mode may cause nascent coagulum or blockage 175 to form.

Fig. 7D shows a view of forming blockage 175 in fourth step 196. The blockage may include charred blood, vessel wall portions, and other components. In some cases, the blockage may form around the tip of the conductive element 140. As described above, operation of the system or device in resistive mode may start to fully occlude the body vessel 112. If the device is in a resistive heating mode, the coil 140 itself will rise in temperature, and direct contact with the vessel wall 112 may be advantageous to achieve faster and more complete occlusion.

Fig. 7E illustrates the withdrawal step 198 after ablation has been achieved. Inflatable element or expandable balloon 149 is deflated to its contracted state 155, and the balloon catheter/ablation device system is withdrawn in proximal direction 181. The flow of blood 129 now proceeds from upstream direction 179 to the treatment site, where it is now stopped at the complete blockage 177, rather than by the expandable balloon 149.

Alternatives to the illustrated embodiment are also contemplated. For instance, the occlusion device or balloon catheter need not be deployed coaxial with or integral with the portion of the ablation device which contains the electrical components. Instead, the balloon catheter may be deployed at a position substantially upstream of the treatment site, and the ablation system may be introduced at a second, downstream site percutaneously, in order to minimize the effective diameter of the system.

As mentioned previously, with RF signal, over time blood in the vessel undergoing ablation may start to char in the local zone of the RF signal. This charring may cause the blood to coagulate on the conductive element, such as the electrode tip or conductive coil, inhibiting the ability of the coil to deliver a sufficient RF signal for vessel ablation. In some cases, this may occur as the ablation treatment is reaching its conclusion, causing the coagulum, blockage, or clot to adhere to the tip or coil. When this occurs, there is a chance that the clot will adhere so strongly that withdrawal of the tip will cause the clot to break free of the vessel wall at the ablation site and be withdrawn with the ablation device. If this occurs, recanalization will occur and blood will readily flow through the lumen of the vessel to be ablated, or even out of the vessel if damage to the vessel wall has been extensive.

One way to prevent such adhesion is to apply a nonstick or low-friction coating to the portion of the conductive element where adhesion is likely to occur. This would allow for withdrawal of the tip without dislodging the clot or blockage. However, many such coatings are insulators of electricity, and their application would render the device ineffective to provide energy to the ablation site. Furthermore, certain coatings are also prone to being damaged by heat.

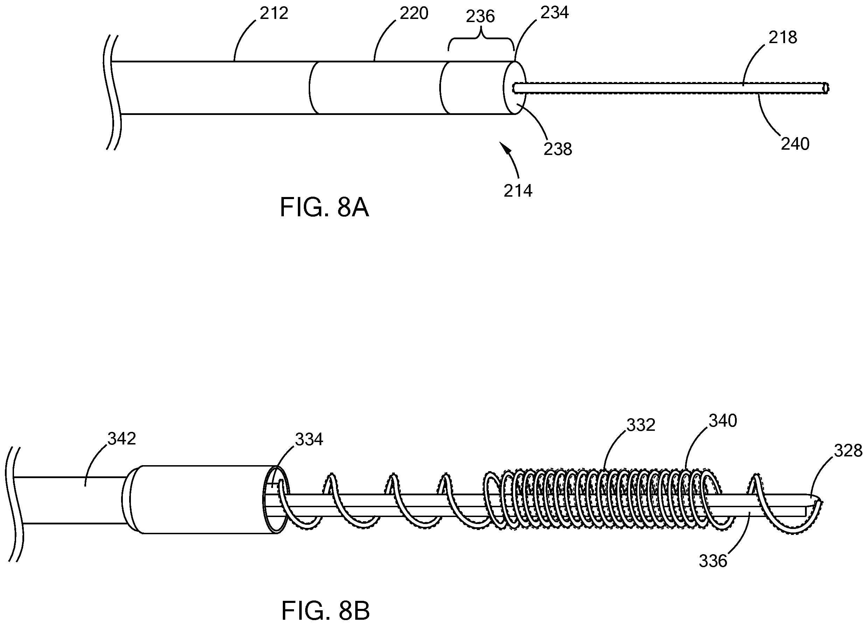

Fig. 8A and 8B illustrate embodiments of devices which have had low-friction coatings applied to portions of their conductive elements. The coatings are dielectric coatings which are thin enough to permit capacitive coupling and thereby permit the device to function. In capacitive coupling, the alternating current in the coil, when separated by the insulative or dielectric barrier, causes heating in the blood, without the flow of electrons through the barrier. In some applications, such as those involving microwave energy which transmit energy in the high megahertz to gigahertz range, a low friction coating is simply applied, as the transmission of energy can occur through even a relatively thick coating. In an RF device, this is much more of a technical challenge. However, in cases of small vessel ablation, it may be advantageous to work with RF instead of microwave radiation, as microwave energy heats in a spread-out fashion and at high tissue depth, and may therefore affect tissues which are not the targets of the procedure. Additionally, RF ablation may be achieved with a relatively smaller device, and provides a more focused energy dosage in a smaller space.

Fig. 8A illustrates a typical RF ablation device 214, which has conductive element 218 extending from sleeve 234. Conductive element 218 is coated with dielectric coating 240. In the embodiment shown, the dielectric coating 240 covers conductive element 218 in its entirety, but other embodiments in which just the distal half, or the distal third, or any other fraction of the distal tip are coated are envisioned. This coating 240, in certain embodiments, may be directly disposed on the conductive element 218.

In one embodiment, the coating 240 is a coating of a polymer. In a specific embodiment, the polymer is parylene C. Parylene C is a dielectric coating which can be coated onto materials by a variety of methods, such as vapor deposition, and can be done in very thin layers while still maintaining a pinhole-free layer. In conjunction with a tip of the present disclosure, the layer of parylene C may range from being about 1.5 nanometers thick to about 1 micron thick. In a particular embodiment, the layer of parylene C may be about 0.01 to about 0.5 micron thick, or about 0.05 micron thick to about 0.25 micron thick, or about 0.1 micron thick to about 0.2 micron thick, or about 0.1 micron thick, or about 0.15 micron thick. In the case of a 0.5 mm diameter conductive element, a 0.1 micron coating will produce an impedance of about 70 ohm at the frequency of about 500 kilohertz.

In the case of RF device 214, face 238 of sleeve 234 may be used to push against the clot during withdrawal. Aided by the low-friction coating 240, the pushing action will improve ease of withdrawal. Optionally, outer sleeve 212 may be provided to enclose the conductive portion of the assembly during withdrawal.

Fig. 8B shows an alternative embodiment of a device having a coated conductive element. In this case, coil 332 is coated with dielectric coating layer 340. In the embodiment shown, the dielectric coating 340 covers conductive element 332 in its entirety, but other embodiments in which just the distal half, or the distal third, or any other fraction of the distal tip are coated are envisioned. The device of Fig. 8B may have both an RF and a resistive heating mode. In this case, an additional advantage of the coating will be realized during resistive heating; namely, that the direct current (or galvanic current) component of the energy will be minimized or eliminated. Nerve responses may be provoked by direct current so elimination is beneficial.

It should be understood that the foregoing relates to exemplary embodiments of the disclosure and that modifications may be made without departing from the scope of the following claims. While the disclosure has been described with respect to certain embodiments it will be appreciated that modifications and changes may be made by those skilled in the art.

## Claims

1. A device to heat a body vessel in a body, the device comprising:

   a coil being electrically conductive for positioning adjacent the treatment site, the coil including a resistive part, the device having a return path, the device being configured to operate in a radiofrequency mode and a resistive mode;
   means for occluding the body vessel at a position upstream of the treatment site such that the treatment site is substantially free of fluid flow; and
   a control unit adapted for:

   transmitting a radiofrequency signal from the coil to the return electrode to heat the body vessel, the resistive part being at a first temperature, the device being in the radiofrequency mode;
   detecting at least one change in the coil; and
   transmitting a current through the coil when the at least one change is detected to heat the resistive part to a second temperature greater than the first temperature and sufficient to occlude the body vessel, the device being in the resistive mode.

2. The device of claim 1, wherein said means for occluding the body vessel comprises an expandable balloon of a balloon catheter, the balloon catheter comprising a catheter body having a first end and extending to a second end, the catheter body defining a central lumen therethrough.

3. The device of claim 2, wherein the balloon catheter is adapted to be introduced percutaneously at a first upstream site to provide occlusion, and the coil is adapted to be introduced at a second downstream site to provide ablation.

4. The device of any one of the preceding claims, wherein the device comprises a support comprising a proximal end and extending to a distal end, the support defining a longitudinal axis, the coil being disposed about the support and having a first end being disposed between the proximal and distal ends of the support, the coil extending from the first end to a second end disposed at the distal end of the support, at least a portion of the support being disposed coaxially within the central lumen of the balloon catheter.

5. The device of claim 4, wherein the support is formed integrally with the balloon catheter.

6. The device of claim 4, wherein the support and the balloon catheter are formed as separate elements.

7. The device of any one of the preceding claims, wherein transmitting a radiofrequency signal comprises transmitting the radiofrequency signal in a monopolar mode or a bipolar mode.

8. The device of any one of the preceding claims, the at least one change being selected from the group consisting of a change in impedance, a change in temperature, a change in time, a change in current, and a change in resistance.

9. The device of any one of the preceding claims, the device comprising:

a balloon catheter comprising a catheter body having a first end and extending to a second end, the catheter body defining a central lumen therethrough, the balloon catheter having an expandable balloon disposed circumferentially about the catheter body;
a support comprising a proximal end and extending to a distal end, the support defining a longitudinal axis, the support being disposed through the central lumen;
said coil being disposed about the support and being electrically conductive, the coil having a first coil end being disposed between the proximal and distal ends, the coil extending from the first coil end to a second coil end disposed at the distal end of the support, the coil extending beyond the second end of the balloon catheter; and
said control unit being operatively coupled to the first end and the second end, the control unit having a signal generator and a power supply, the signal generator operable to transmit a radiofrequency signal from the coil to the body vessel when the device is in a radiofrequency mode, the resistive part being at a first temperature in the radiofrequency mode, the power supply operable to transmit a current through the coil when the device is in a resistive mode, the current heating the resistive part to a second temperature greater than the first temperature and sufficient to occlude the body vessel.

10. The device of claim 9, wherein the support has a distal segment about which the coil is disposed, the distal segment having an outer diameter that distally decreases to form a distal taper.

11. The device of claim 9 or claim 10, further comprising a first wire and a second wire, the first wire being electrically coupled to the control unit and extending from the control unit and along the longitudinal axis to the first end, the first wire being attached to the first end, the second wire also being electrically coupled to the control unit and extending from the control unit and along the longitudinal axis to the second end, the second wire being attached to the second end, the control unit, the first wire, the coil, and second wire forming a first circuit operable in the resistive mode.

12. The device of claim 11, further comprising an insulator disposed about the first and second wires to electrically insulate them from the rest of the device.

13. The device of any one of claim 9 to claim 12, further comprising a sheath having a first sheath end and extending to a second sheath end, the sheath forming an inner lumen therethrough from the first sheath end to the second sheath end, the support being slidably disposed within the inner lumen, the outer sheath being sized to fit within the central lumen of the balloon catheter

14. The device of claim 9, wherein the outer sheath is formed integrally with the balloon catheter or is formed as a separate component from the balloon catheter.

15. The device of any one of claim 9 to claim 14, wherein the catheter body comprises at least one inflation lumen, the expandable balloon being in fluid connection with the catheter body via the inflation lumen.

**Patentansprüche**

1. Vorrichtung zum Wärmen eines Körpergefässes in einem Körper, wobei die Vorrichtung umfasst:

eine Spule, die elektrisch leitfähig ist, um in der Nähe des Behandlungsorts platziert zu werden, wobei die Spule ein ohmsches Bauteil enthält, wobei die Vorrichtung eine Rückleitung aufweist, wobei die Vorrichtung konfiguriert ist, um in einem Hochfrequenzmodus und einem ohmschen Modus zu funktionieren;
ein Element zum Verschließen des Körpergefässes an einer zum Behandlungsort vorgeschalteten Position, sodass der Behandlungsort im Wesentlichen frei von einer Flüssigkeitsströmung ist; und
eine Steuereinheit, die geeignet ist zum:

Übertragen eines Hochfrequenzsignals von der Spule zur Rückleitelektrode, um das Körpergefäß zu wärmen, wobei das ohmsche Bauteil auf einer ersten Temperatur liegt, wobei sich die Vorrichtung im Hochfrequenzmodus befindet;

Erkennen mindestens einer Änderung in der Spule; und

Übertragen eines Stroms durch die Spule, wenn die mindestens eine Änderung erkannt wird, um das ohmsche Bauteil auf eine zweite Temperatur zu wärmen, die höher als die erste Temperatur ist und ausreichend ist, um das Körpergefäß zu verschließen, wobei sich die Vorrichtung im ohmschen Modus befindet.

2. Vorrichtung nach Anspruch 1, wobei das Element zum Verschließen des Körpergefässes einen ausdehnbaren Ballon eines Ballonkatheters umfasst, wobei der Ballonkatheter einen Katheterkörper umfasst, der ein erstes Ende aufweist und sich bis zu einem zweiten Ende erstreckt, wobei durch den Katheterkörper ein zentrales Lumen definiert ist.

3. Vorrichtung nach Anspruch 2, wobei der Ballonkatheter geeignet ist, um an einem ersten vorgeschalteten Ort perkutan eingeführt zu werden, um einen Verschluss bereitzustellen, und wobei die Spule geeignet ist, um an einem zweiten nachgeschalteten Ort eingeführt zu werden, um eine Ablation bereitzustellen.

4. Vorrichtung nach einem der vorhergehenden Ansprüche, wobei die Vorrichtung einen Träger umfasst, der ein proximales Ende umfasst und sich zu einem distalen Ende erstreckt, wobei der Träger eine Längsachse definiert, wobei die Spule um den Träger angeordnet ist und ein erstes Ende aufweist, das zwischen dem proximalen und dem distalen Ende des Trägers angeordnet ist, wobei sich die Spule von dem ersten Ende zu einem zweiten Ende erstreckt, das an dem distalen Ende des Trägers angeordnet ist, wobei mindestens ein Abschnitt des Trägers koaxial innerhalb des Lumens des Ballonkatheters angeordnet ist.

5. Vorrichtung nach Anspruch 4, wobei der Träger als integraler Bestandteil des Ballonkatheters gebildet ist.

6. Vorrichtung nach Anspruch 4, wobei der Träger und der Ballonkatheter als getrennte Elemente gebildet sind.

7. Vorrichtung nach einem der vorhergehenden Ansprüche, wobei das Übertragen eines Hochfrequenzsignals ein Übertragen des Hochfrequenzsignals in einem monopolaren Modus oder in einem bipolaren Modus umfasst.

8. Vorrichtung nach einem der vorhergehenden Ansprüche, wobei die mindestens eine Änderung aus der Gruppe ausgewählt wird, die aus einer Impedanzänderung, einer Temperaturänderung, einer Zeitdaueränderung, einer Stromänderung und einer Widerstandsänderung besteht.

9. Vorrichtung nach einem der vorhergehenden Ansprüche, wobei die Vorrichtung umfasst:

einen Ballonkatheter, der einen Katheterkörper umfasst, der ein erstes Ende aufweist und sich bis zu einem zweiten Ende erstreckt, wobei durch den Katheterkörper ein zentrales Lumen definiert ist, wobei der Ballonkatheter einen ausdehnbaren Ballon aufweist, der um den Umfang des Katheterkörpers angeordnet ist;

einen Träger, der ein proximales Ende umfasst und sich zu einem distalen Ende erstreckt, wobei der Träger eine Längsachse definiert, wobei der Träger durch das Lumen angeordnet ist;

wobei die Spule um den Träger angeordnet ist und elektrisch leitfähig ist, wobei die Spule ein erstes Spulenende aufweist, das zwischen dem proximalen und dem distalen Ende angeordnet ist, wobei sich die Spule von dem ersten Ende zu einem zweiten Ende erstreckt, das an dem distalen Ende des Trägers angeordnet ist, wobei sich die Spule über das zweite Ende des Ballonkatheters hinaus erstreckt; und

wobei die Steuereinheit funktionsfähig mit dem ersten Ende und dem zweiten Ende verbunden ist, wobei die Steuereinheit einen Signalgenerator und eine Stromversorgung aufweist, wobei der Signalgenerator funktionsfähig ist, um ein Hochfrequenzsignal von der Spule an das Körpergefäß zu übertragen, wenn sich die Vorrichtung in einem Hochfrequenzmodus befindet, wobei das ohmsche Bauteil in dem Hochfrequenzmodus auf einer ersten Temperatur liegt, wobei die Stromversorgung funktionsfähig ist, um einen Strom durch die Spule zu übertragen, wenn sich die Vorrichtung in einem ohmschen Modus befindet, wobei der Strom das ohmsche Bauteil auf eine zweite Temperatur aufwärmt, die höher als die erste Temperatur ist und ausreichend ist, um das Körpergefäß zu verschließen.

10. Vorrichtung nach Anspruch 9, wobei der Träger ein distales Segment aufweist, um das die Spule angeordnet ist, wobei das distale Segment einen Außendurchmesser aufweist, der in distaler Richtung abnimmt, um eine distale Verjüngung zu bilden.

**11.** Vorrichtung nach Anspruch 9 oder Anspruch 10, die außerdem einen ersten Draht und einen zweiten Draht umfasst, wobei der erste Draht mit der Steuereinheit elektrisch verbunden ist und sich von der Steuereinheit und entlang der Längsachse zu dem ersten Ende erstreckt, wobei der erste Draht an dem ersten Ende befestigt ist, wobei der zweite Draht auch mit der Steuereinheit elektrisch verbunden ist und sich von der Steuereinheit und entlang der Längsachse zu dem zweiten Ende erstreckt, wobei der zweite Draht an dem zweiten Ende befestigt ist, wobei die Steuereinheit, der erste Draht, die Spule und der zweite Draht einen ersten Stromkreis bilden, der im ohmschen Modus funktionsfähig ist.

**12.** Vorrichtung nach Anspruch 11, die außerdem ein Isolationselement umfasst, das um den ersten und den zweiten Draht angeordnet ist, um sie von dem Rest der Vorrichtung elektrisch zu isolieren.

**13.** Vorrichtung nach einem der Ansprüche 9 bis 12, die außerdem eine Hülle umfasst, die ein erstes Hüllenende aufweist und sich zu einem zweiten Hüllenende erstreckt, wobei durch die Hülle vom ersten Hüllenende zum zweiten Hüllenende ein Innenlumen gebildet wird, wobei der Träger verschiebbar in dem Innenlumen angeordnet ist, wobei die Außenhülle dimensioniert ist, um in das zentrale Lumen des Ballonkatheters zu passen.

**14.** Vorrichtung nach Anspruch 9, wobei die Außenhülle als integraler Bestandteil des Ballonkatheters gebildet ist oder als eine von dem Ballonkatheter getrennte Komponente gebildet ist.

**15.** Vorrichtung nach einem der Ansprüche 9 bis 14, wobei der Katheterkörper mindestens ein Aufblaslumen umfasst, wobei der ausdehnbare Ballon über das Aufblaslumen in einer Strömungsverbindung mit dem Katheterkörper steht.

**Revendications**

**1.** Dispositif permettant de chauffer un vaisseau corporel dans un organisme, le dispositif comprenant :

une bobine étant électroconductrice pour un positionnement adjacent au site de traitement, la bobine comprenant une partie résistive, le dispositif ayant un trajet de retour, le dispositif étant conçu pour fonctionner dans un mode radiofréquence et un mode résistif ;
des moyens pour occlure le vaisseau corporel au niveau d'une position en amont du site de traitement de sorte que le site de traitement soit sensiblement dépourvu d'un écoulement de fluide ; et
une unité de commande conçue pour :

transmettre un signal radiofréquence depuis la bobine vers l'électrode de retour afin de chauffer le vaisseau corporel, la partie résistive étant à une première température, le dispositif étant dans le mode radiofréquence ;
détecter au moins un changement dans la bobine ; et
transmettre un courant à travers la bobine lorsque l'au moins un changement est détecté pour chauffer la partie résistive à une seconde température supérieure à la première température et suffisante pour occlure le vaisseau corporel, le dispositif étant dans le mode résistif.

**2.** Dispositif selon la revendication 1, dans lequel lesdits moyens pour occlure le vaisseau corporel comprennent un ballonnet déployable d'un cathéter à ballonnet, le cathéter à ballonnet comprenant un corps de cathéter ayant une première extrémité et s'étendant vers une seconde extrémité, le corps de cathéter délimitant une lumière centrale à travers celui-ci .

**3.** Dispositif selon la revendication 2, dans lequel le cathéter à ballonnet est conçu pour être introduit par voie cutanée au niveau d'un premier site en amont pour permettre l'occlusion, et la bobine est conçue pour être introduite au niveau d'un second site en aval pour permettre l'ablation.

**4.** Dispositif selon l'une quelconque des revendications précédentes, le dispositif comprenant un support comprenant une extrémité proximale et s'étendant vers une extrémité distale, le support délimitant un axe longitudinal, la bobine étant disposée autour du support et ayant une première extrémité qui est disposée entre les extrémités proximale et distale du support, la bobine s'étendant depuis la première extrémité vers une seconde extrémité disposée au niveau de l'extrémité distale du support, au moins une partie du support étant disposée de façon coaxiale à l'intérieur de la lumière centrale du cathéter à ballonnet.

**5.** Dispositif selon la revendication 4, dans lequel le support est formé d'un seul tenant avec le cathéter à ballonnet.

**6.** Dispositif selon la revendication 4, dans lequel le support et le cathéter à ballonnet sont formés en tant qu'éléments distincts.

**7.** Dispositif selon l'une quelconque des revendications précédentes, dans lequel la transmission d'un signal radiofréquence consiste à transmettre le signal radiofréquence dans un mode monopolaire ou un mode bipolaire.

**8.** Dispositif selon l'une quelconque des revendications précédentes, l'au moins un changement étant choisi dans le groupe constitué par un changement d'impédance, un changement de température, un changement de temps, un changement de courant et un changement de résistance.

**9.** Dispositif selon l'une quelconque des revendications précédentes, le dispositif comprenant :

un cathéter à ballonnet comprenant un corps de cathéter possédant une première extrémité et s'étendant vers une seconde extrémité, le corps de cathéter délimitant une lumière centrale à travers celui-ci, le cathéter à ballonnet possédant un ballonnet déployable disposé circonférentiellement autour du corps de cathéter ;
un support comprenant une extrémité proximale et s'étendant vers une extrémité distale, le support délimitant un axe longitudinal, le support étant disposé à travers la lumière centrale ;
ladite bobine étant disposée autour du support et étant électroconductrice, la bobine ayant une première extrémité de bobine qui est disposée entre les extrémités proximale et distale, la bobine s'étendant depuis la première extrémité de bobine vers une seconde extrémité de bobine qui est disposée au niveau de l'extrémité distale du support, la bobine s'étendant au-delà de la seconde extrémité du cathéter à ballonnet ; et
ladite unité de commande étant accouplée fonctionnellement à la première extrémité et à la seconde extrémité, l'unité de commande ayant un générateur de signaux et une alimentation électrique, le générateur de signaux pouvant être mis en oeuvre pour transmettre un signal radiofréquence depuis la bobine vers le vaisseau corporel lorsque le dispositif est dans un mode radiofréquence, la partie résistive étant à une première température dans le mode radiofréquence, l'alimentation électrique pouvant être mise en oeuvre pour transmettre un courant à travers la bobine lorsque le dispositif est dans un mode résistif, le courant chauffant la partie résistive à une seconde température supérieure à la première température et suffisante pour occlure le vaisseau corporel.

**10.** Dispositif selon la revendication 9, dans lequel le support possède un segment distal autour duquel est disposée la bobine, le segment distal ayant un diamètre externe qui diminue dans le sens distal pour former un cône distal.

**11.** Dispositif selon la revendication 9 ou la revendication 10, comprenant en outre un premier fil et un second fil, le premier fil étant couplé électriquement à l'unité de commande et s'étendant depuis l'unité de commande et le long de l'axe longitudinal vers la première extrémité, le premier fil étant fixé à la première extrémité, le second fil étant également couplé électriquement à l'unité de commande et s'étendant depuis l'unité de commande et le long de l'axe longitudinal vers la seconde extrémité, le second fil étant fixé à la seconde extrémité, l'unité de commande, le premier fil, la bobine et le second fil formant un premier circuit pouvant être mis en oeuvre dans le mode résistif.

**12.** Dispositif selon la revendication 11, comprenant en outre un isolateur disposé autour des premier et second fils afin de les isoler électriquement du reste du dispositif.

**13.** Dispositif selon l'une quelconque des revendications 9 à 12, comprenant en outre une gaine ayant une première extrémité de gaine et s'étendant vers une seconde extrémité de gaine, la gaine formant une lumière interne à travers celle-ci depuis la première extrémité de gaine vers la seconde extrémité de gaine, le support étant disposé en coulissement à l'intérieur de la lumière interne, la gaine externe étant dimensionnée pour s'ajuster dans la lumière centrale du cathéter à ballonnet.

**14.** Dispositif selon la revendication 9, dans lequel la gaine externe est formée d'un seul tenant avec le cathéter à ballonnet ou est formée en tant qu'élément distinct du cathéter à ballonnet.

**15.** Dispositif selon l'une quelconque des revendications 9 à 14, dans lequel le corps de cathéter comprend au moins une lumière de gonflage, le ballonnet déployable étant en raccord fluidique avec le corps de cathéter par l'intermédiaire de la lumière de gonflage.

FIG. 1

FIG. 2A

FIG. 2B

FIG. 3

EP 3 210 558 B1

EP 3 210 558 B1

ESU
RF
Generator

FIG. 4

FIG. 5

FIG. 6

FIG. 7A 190

FIG. 7B 192

FIG. 7C 194

FIG. 7D 196

FIG. 7E 198

EP 3 210 558 B1

FIG. 8A

FIG. 8B

EP 3 210 558 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0707830 A1

- EP 2939629 A2